# EUROPEAN PATENT SPECIFICATION

(11) **EP 3 505 489 B1**
(45) Date of publication and mention of the grant of the patent: **25.03.2020**
(21) Application number: 17843275.3
(22) Date of filing: 21.07.2017
(51) Int. Cl.: B81B 1/00, C12M 1/34, C12M 1/00

(54) **SINGLE-PARTICLE CAPTURING APPARATUS, SINGLE-PARTICLE CAPTURING SYSTEM, AND METHOD FOR CAPTURING SINGLE-PARTICLE**
EINZELPARTIKELERFASSUNGSVORRICHTUNG, EINZELPARTIKELERFASSUNGSSYSTEM UND VERFAHREN ZUR ERFASSUNG VON EINZELPARTIKELN
APPAREIL DE CAPTURE DE PARTICULE UNIQUE, SYSTÈME DE CAPTURE DE PARTICULE UNIQUE ET PROCÉDÉ DE CAPTURE DE PARTICULE UNIQUE

(30) Priority: 23.08.2016 JP 2016162644
(43) Date of publication of application: 03.07.2019
(73) Proprietor: Sony Corporation, Tokyo 108-0075 (JP)
(72) Inventor: KOJIMA, Kensuke, Tokyo 108-0075 (JP); MASUHARA, Shin, Tokyo 108-0075 (JP); WATANABE, Toshio, Tokyo 108-0075 (JP)
(74) Representative: D Young & Co LLP
(86) International application number: PCT/JP2017/026383
(87) International publication number: WO 2018/037788

(56) References cited:
- EP-A1- 1 340 809
- EP-A1- 1 762 608
- WO-A1-2015/087369
- JP-A- S61 501 126
- JP-A- 2005 506 083
- JP-A- 2009 123 633
- JP-A- 2009 195 160
- JP-A- 2012 065 649
- US-A1- 2013 078 163

## Description

### TECHNICAL FIELD

The present invention relates to a single-particle capturing apparatus, a single-particle capturing system, and a single-particle capturing method.

### BACKGROUND ART

In recent years, a technology in which a cell is captured alone as typified by flow cytometry or the like, has been developed. The cell, after being captured alone, is used for analysis or culture.

As a method for capturing a single cell, for example, a technology described in Patent Document 1 has been developed. Patent Document 1 discloses a single-cell capturing array in which a well large enough for a single cell is formed on a flow channel through which a cell-containing sample flows and the single cell is captured in the well while the cell-containing sample flows (Figs. 1 to 8). Furthermore, a structure in which a cell is sucked through a slit provided on the well is disclosed (Figs. 23, 25 and the like).

### CITATION LIST

### PATENT DOCUMENT

Patent Document 1: US 2013/0078163 A

### SUMMARY OF THE INVENTION

### PROBLEMS TO BE SOLVED BY THE INVENTION

However, with the structure of the flow channel of the aforementioned Patent Document 1, a phenomenon occurs in which, even after the cell is trapped in the well, another cell is sucked to the well and adhered to the captured cell, resulting in accumulation of a plurality of cells.

One of possible causes is an excessive number of supplied cells. Once the cells are accumulated, an accumulation starts snowballing at the accumulation point and grows until the flow channel is clogged. The cells once accumulated are difficult to move, resulting in inconvenient operability.

### SOLUTIONS TO PROBLEMS

In order to solve the aforementioned problem, the present technology provides a single-particle capturing apparatus including a flow channel on a substrate, a wave structure with a mountain portion and a valley portion on the flow channel, and a recess portion at a top portion of the mountain portion, the recess portion including a draw-in passage.

The depth of the recess portion can be equal to or less than the particle diameter of a particle to be captured, and the diameter of the recess portion can be a size equal to or more than one time and less than two times of the particle diameter of the particle to be captured.

Furthermore, the height from the valley portion to the mountain portion can be equal to or larger than the particle diameter of the particle to be captured, the pitch between the mountain portions can be a length equal to or more than 2 times and equal to or less than 20 times of the particle diameter of a particle to be captured, and the channel width of the flow channel can be relatively small at the mountain portion and relatively large at the valley portion.

Furthermore, the draw-in passage can make communication between the recess portion and the outside.

Moreover, a plurality of mountain portions and valley portions may be aligned on the bottom surface of the flow channel.

Moreover, the flow channel and the wave structure may be curved or bent.

For example, the flow channel and the wave structure are curved in a U-shape, and an inner side of the U-shape is the outside.

The present technology can provide a single-particle capturing system including
a single-particle capturing unit including
a flow channel on a substrate, a wave structure with a mountain portion and a valley portion on the flow channel, and a recess portion at a top portion of the mountain portion, the recess portion including a draw-in passage; and
a liquid supply unit.

The flow channel can include a valve. Furthermore, the single-particle capturing system may include a waste liquid unit, a single-particle capturing observing unit, and a liquid supply control unit.

Moreover, the present technology provides a single-particle capturing method in which a specimen containing a particle to be captured is supplied to a single-particle capturing apparatus including
a flow channel on a substrate, the flow channel including a wave structure with a mountain portion and a valley portion, and a recess portion at a top portion of the mountain portion, the recess portion including a draw-in passage, and
the specimen is, while being supplied, sucked from the recess portion to an outside via the draw-in passage such that the particle to be captured is captured.

The single-particle capturing method can include flowing the supplied liquid backward.

### EFFECTS OF THE INVENTION

According to the present technology, one particle can be captured in one recess portion while preventing another particle from being accumulated on a captured particle.

Note that effects described herein are not necessarily limited, but may also be any of those described in the present disclosure.

### BRIEF DESCRIPTION OF DRAWINGS

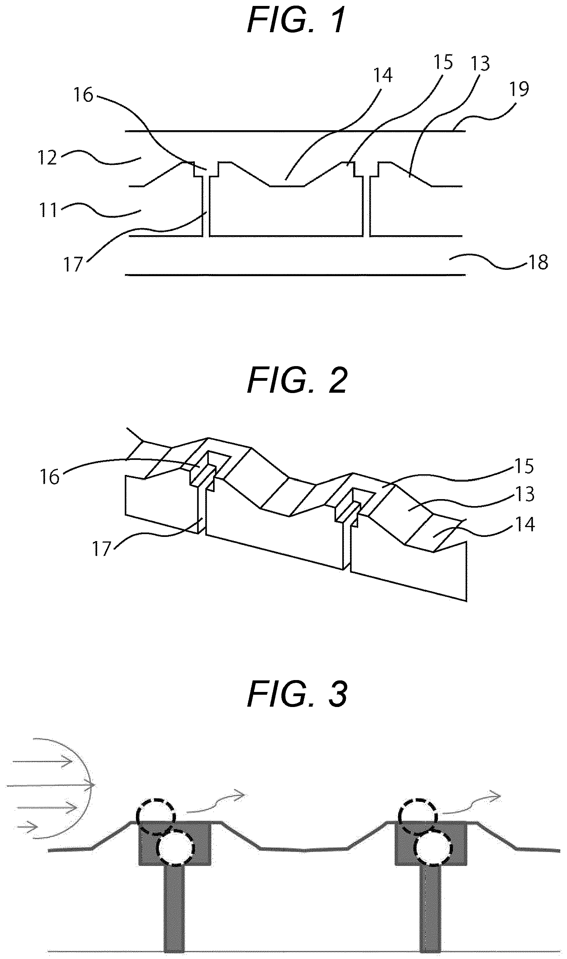

Fig. 1 is a longitudinal sectional view of a single-particle capturing apparatus of the present technology.
Fig. 2 is a perspective view illustrating a mountain portion, a valley portion, a recess portion, and a draw-in passage of a single-particle capturing apparatus of the present technology.
Fig. 3 is a schematic view illustrating a flow of a sample and a state of capturing of particles in a single-particle capturing apparatus of the present technology.

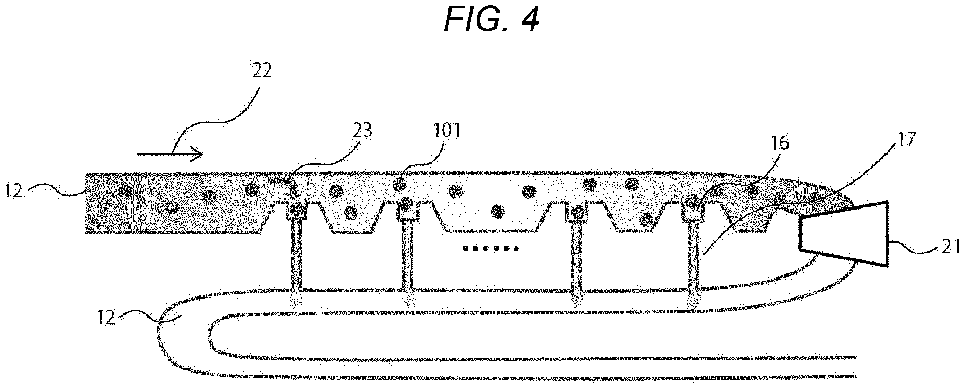

Fig. 4 is a schematic view illustrating a flow of a sample and a state of capturing of particles in a single-particle capturing apparatus of the present technology.

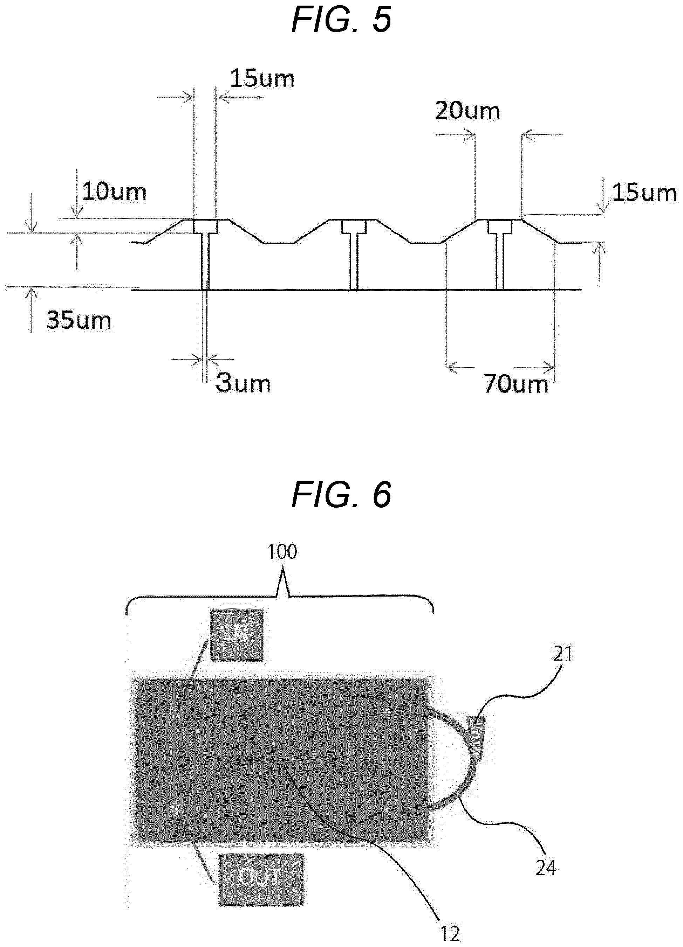

Fig. 5 is a schematic view illustrating an example of a size of a single-particle capturing apparatus of the present technology.
Fig. 6 is a schematic view illustrating an example of a single-particle capturing apparatus of the present technology.

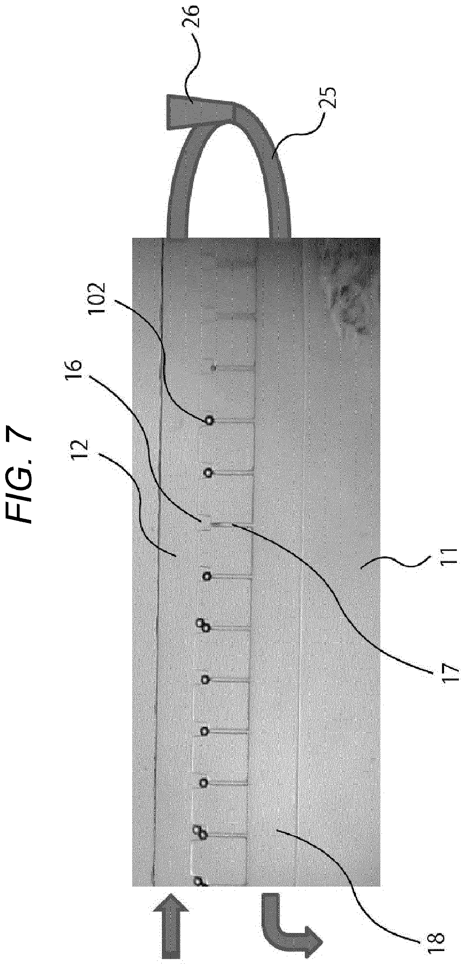

Fig. 7 is a drawing-substitute photograph illustrating a single-particle capturing apparatus of a conventional technology and a state of capturing of particles.

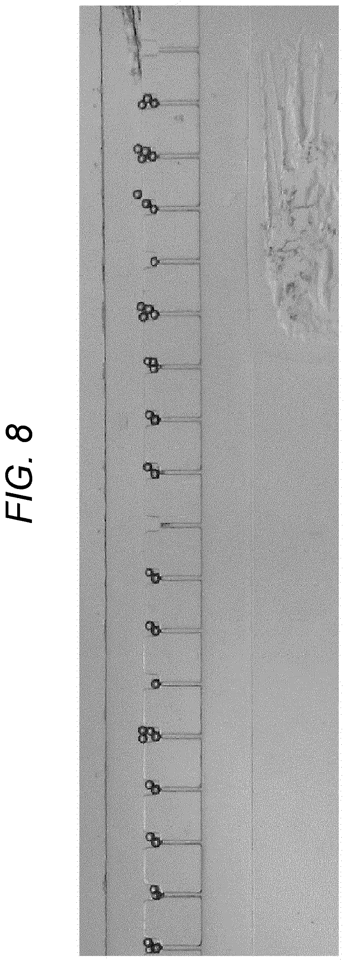

Fig. 8 is a drawing-substitute photograph illustrating a single-particle capturing apparatus of a conventional technology and a state of capturing of particles.

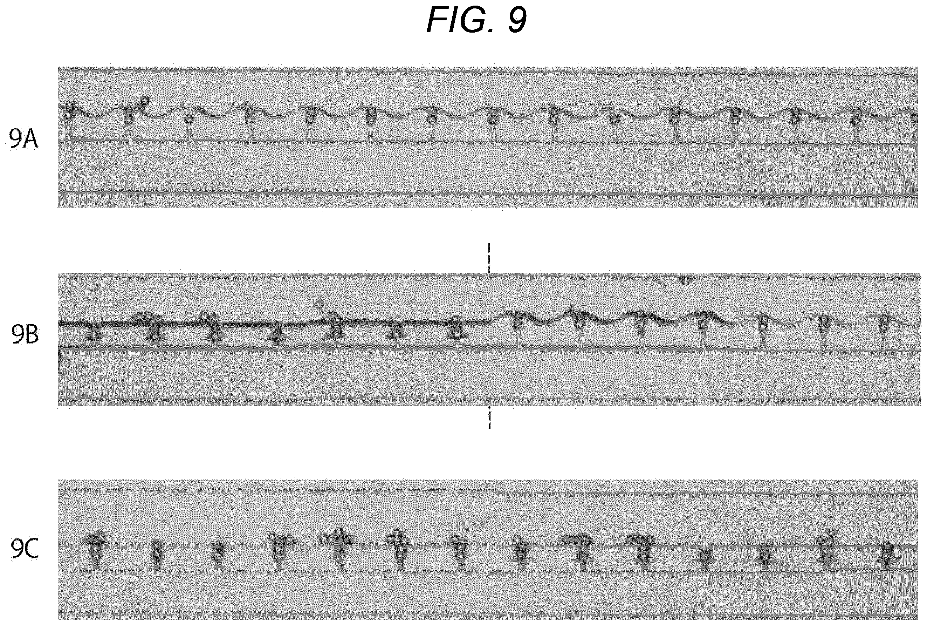

Fig. 9 is drawing-substitute photographs illustrating a single-particle capturing apparatus and a state of capturing of particles.

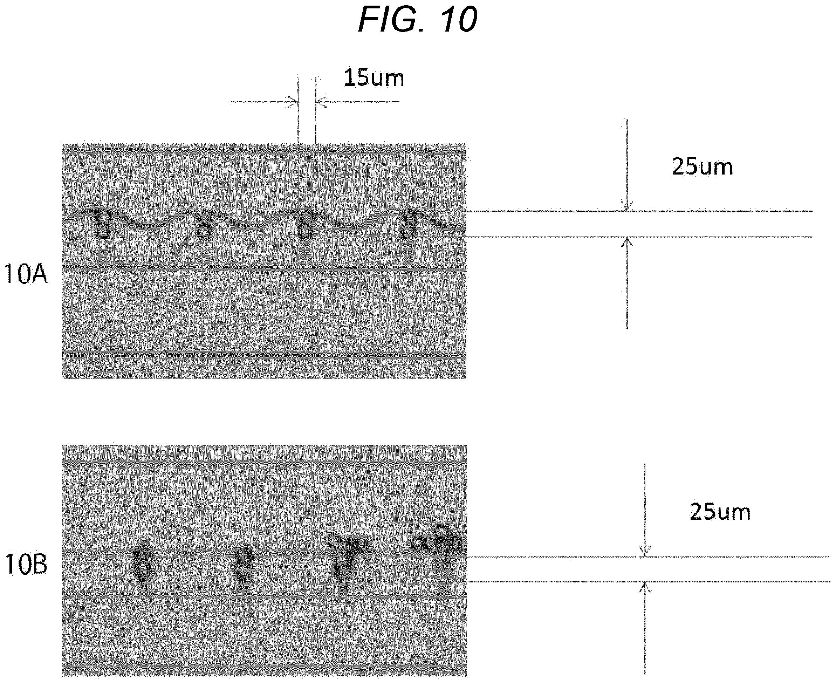

Fig. 10 is drawing-substitute photographs illustrating a single-particle capturing apparatus and a state of capturing of particles.

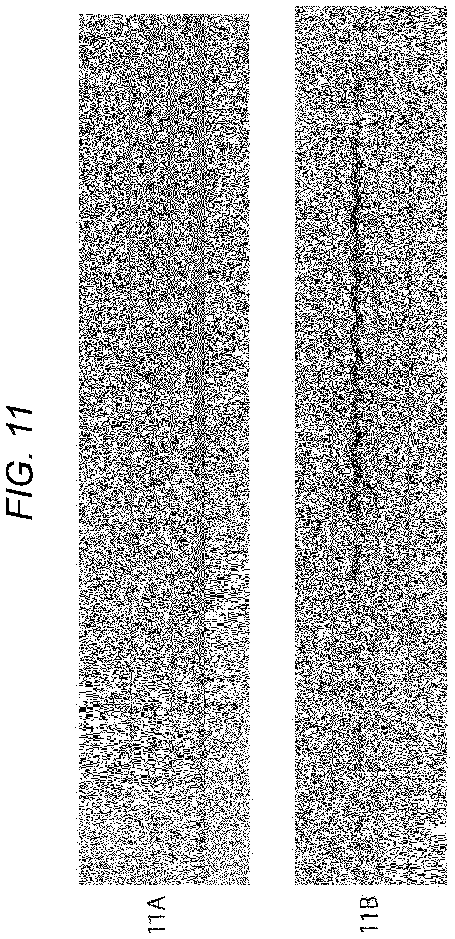

Fig. 11 is drawing-substitute photographs illustrating a single-particle capturing apparatus of the present technology and a state of capturing of particles.

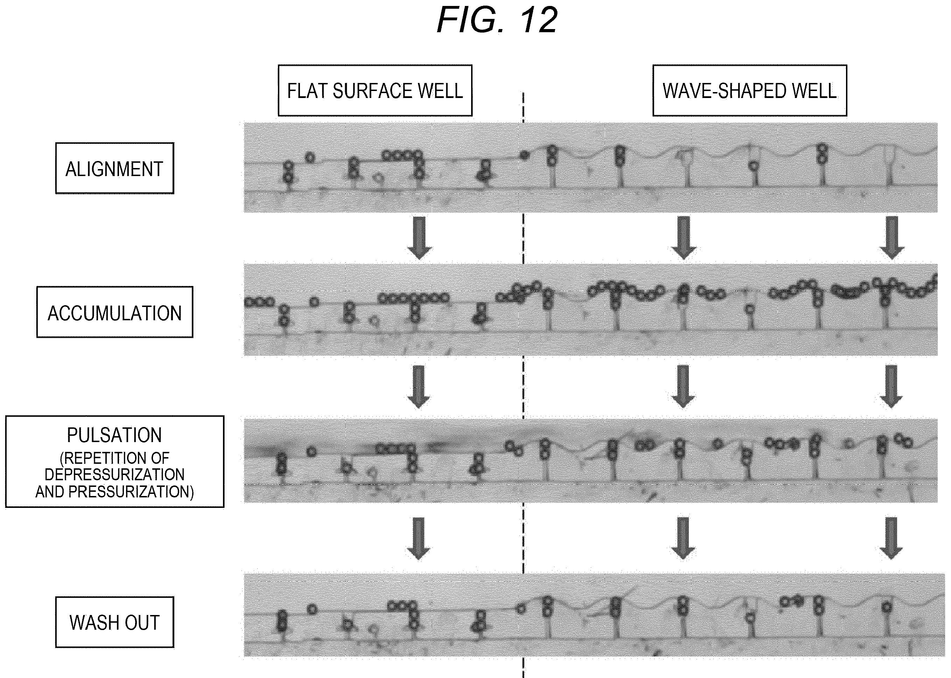

Fig. 12 is a drawing-substitute photograph illustrating a single-particle capturing apparatus of the present technology and a conventional technology and a state of capturing of particles.

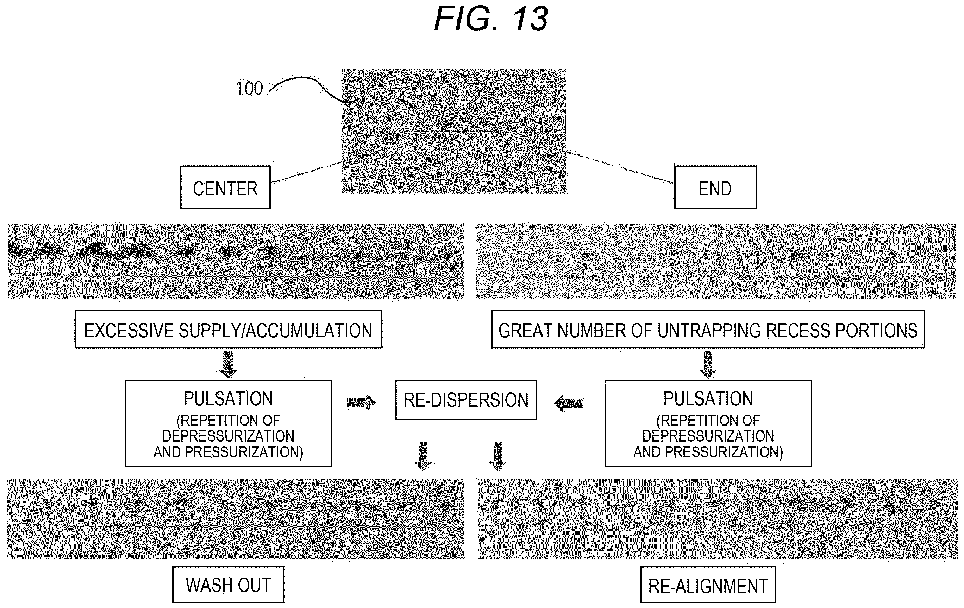

Fig. 13 is a drawing-substitute photograph illustrating a single-particle capturing apparatus of the present technology and a state of capturing of particles.

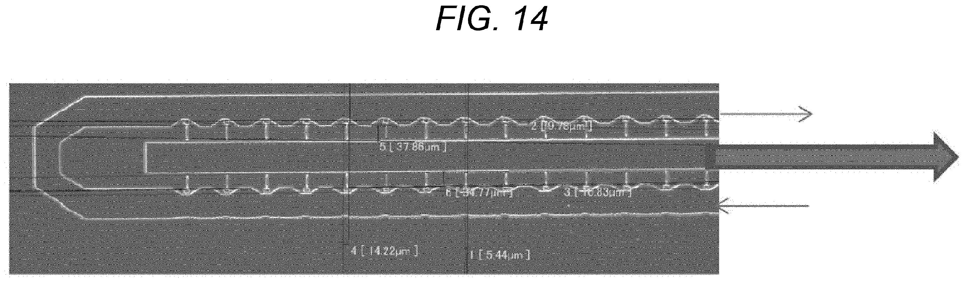

Fig. 14 is a drawing-substitute photograph illustrating an example of a single-particle capturing apparatus of the present technology.

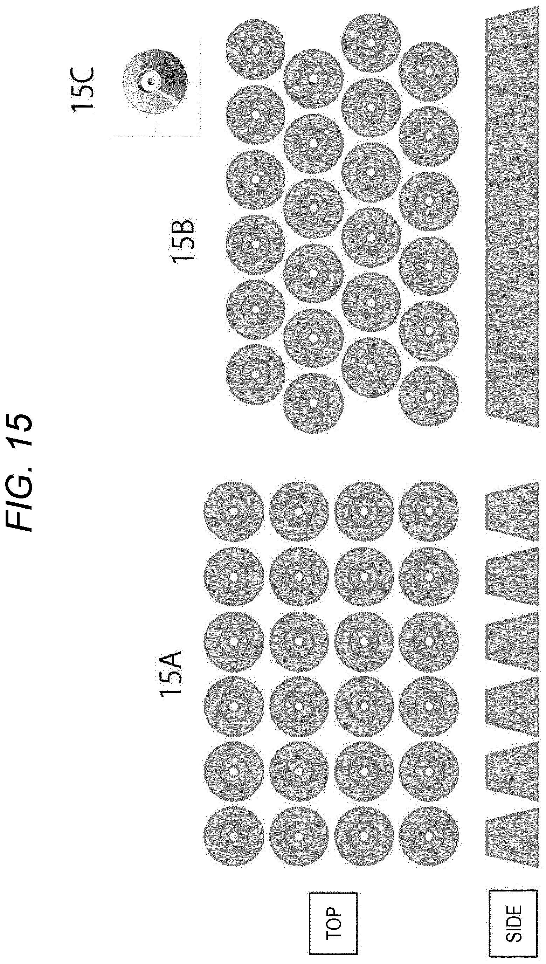

Fig. 15 is schematic views illustrating an example of a single-particle capturing apparatus of the present technology.

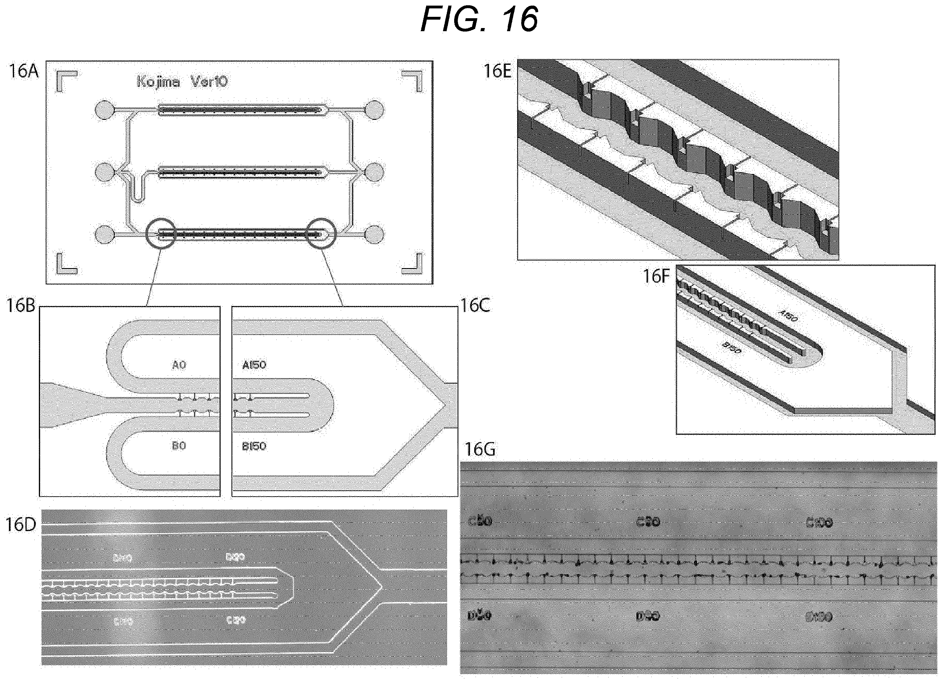

Fig. 16 is diagrams illustrating an example of a single-particle capturing apparatus of the present technology.

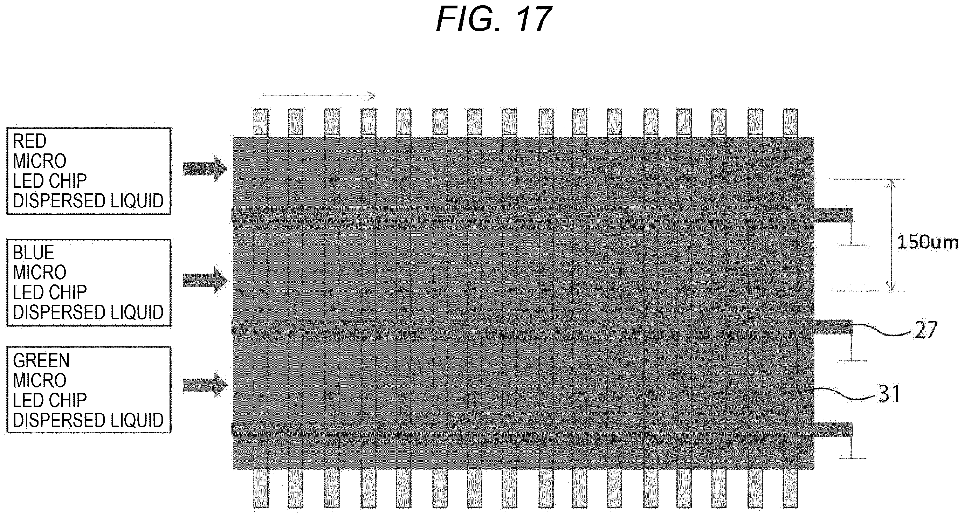

Fig. 17 is a diagram illustrating an example of a single-particle capturing apparatus of the present technology.

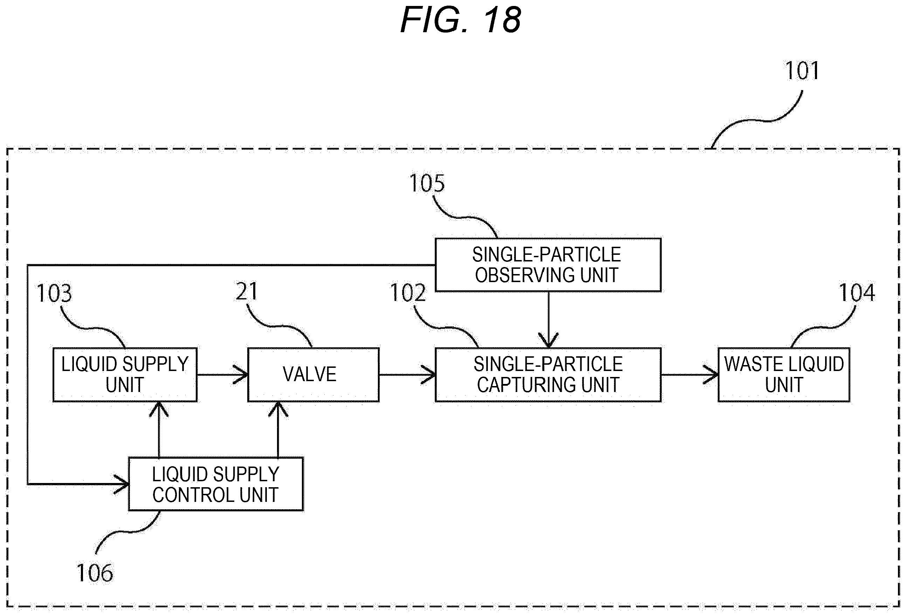

Fig. 18 is a schematic diagram illustrating an example of a single-particle capturing system of the present technology.

### MODE FOR CARRYING OUT THE INVENTION

Preferred aspects for carrying out the present technology are described below. Note that embodiments described below indicate representative embodiments of the present technology, and they do not make the scope of the present technology to be understood narrowly. The description is provided in the order set forth below.
1. Single-particle capturing apparatus
2. Embodiments
   (1) Embodiment 1
   (2) Conventional Technology Example 1
   (3) Conventional Technology Example 2 and Embodiment 2
   (4) Embodiment 3
   (5) Embodiment 4
   (6) Embodiment 5
   (7) Embodiment 6
   (8) Embodiment 7
   (9) Embodiment 8
   (10) Embodiment 9
   (11) Embodiment 10
3. Single-particle capturing system
4. Single-particle capturing method

### <1. Single-particle capturing apparatus>

The type of particles to be captured by a single-particle capturing apparatus of the present technology is not particularly limited. Examples of the type include cells, beads, semiconductor chips, micro bumps as a terminal of a connection portion of a semiconductor, bead-type solar cells, and the like. Furthermore, the size, shape, and the like of particles are also not particularly limited.

Examples of the technical field to which the single-particle capturing apparatus of the present technology can be applied include a hybrid bio/inorganic material, nanohybrid environmental sensor, environmental sensor: sensor array formation technology, light condensing material for solar cell, self-organization arrangement of a chip-shaped component for a high-density package module, a formation technology using a template of a self-organization pattern of a cyclic recess and protrusion structure with a sub-wavelength (sub µm) size necessary for increased light extraction efficiency of a light emitting device or the like, formation of a quasi-phase matching structure by optical polling of a non-linear organic dye for an organic light switching device, self-organization of a metal or semiconductor nanoparticle for quantum dot memory, a polymeric self-organizing material for nanocrystal memory, and the like.

A description is given below with reference to Figs. 1 and 2.

A single-particle capturing apparatus of the present technology includes a flow channel 12 in a substrate 11.

The substrate 11 is not particularly limited, and includes: resin such as polyethylene, polypropylene, vinyl chloride resin, polystyrene, polyethylene terephthalate, acrylic resin, polycarbonate, fluororesin, polybutylene terephthalate, phenolic resin, melamine resin, epoxy resin, unsaturated polyester resin, and polydimethylsiloxane; glass; metal; and the like.

For the flow channel 12, the width and the height of the flow channel can be determined according to the size, shape, and type of a particle to be captured, or the amount, viscosity or the like of a sample that flows in the flow channel.

A wave structure with a mountain portion 13 and a valley portion 14 is included on the flow channel 12, and a recess portion 16 is formed on a top portion 15 of the mountain portion 13. A particle in the sample is captured in the recess portion 16.

By a wave structure on a bottom surface in the flow channel 12, another cell is prevented from adhering to a cell captured in the recess portion at a top portion of the wave. Therefore, it is possible to prevent accumulation of cells.

Furthermore, as illustrated in Fig. 3, the flow channel 12 including a top surface 19 has a characteristic that a liquid flow of the sample therein is laminar and the flow speed at the center of the flow channel 12 is always faster than the flow speed near a side surface of the flow channel (four arrows at the upper left). Therefore, the flow speed at the top portion 15 becomes faster as a result of providing the recess portion 16 at the wave-shaped top portion 15 of the wave structure. Thus, by providing the recess portion 16 at the top portion 15, it is possible to prevent doublet -- two or more particles enter the recess portion 16 (dotted line circles). That is, it is though that even if the second cell or bead adheres to make doublet, because of faster flow speed, the second and subsequent cells and beads hardly enter by being flown by a central laminar flow. For example, the central laminar flow is faster by about 20% than the general flow speed of the liquid flow.

The recess portion 16 further includes a draw-in passage 17. As illustrated in Fig. 4, the sample moves in a liquid flow direction 22 and moves downstream as a valve 21 is opened. Then, because of the presence of the draw-in passage 17 through which the recess portion 16 communicates with the outside (downstream side of the valve 21), a force of draw-in by positive pressure 23 occurs in a direction from the flow channel 12 side to an outside 18. A particle easily enters the recess portion 16 because of a pressure difference between the inside of the flow channel 12 and the outside. Note that, in Fig. 4, the outside is a flow channel 12 of the downstream side of the valve 21 and is contiguous with the flow channel 12.

Note that the installment of the valve is not limited to the above. For example, a valve for flowing a sample liquid can be installed upstream of the flow channel 12 contiguous with the recess portion 16, and a valve for sucking the sample liquid can be installed downstream.

The shape of the recess portion 16 can be determined in accordance with the shape or the like of a particle to be captured. Examples of the shape of the recess portion 16 include a cylindrical shape, a circular truncated conical shape, an inverted circular truncated conical shape, an elliptic cylindrical shape, an elliptic truncated shape, an inverted elliptic truncated conical shape, a tapered shape, an inverted tapered shape, a polygonal column of a triangular column or a column with more corners, and the like.

Furthermore, the depth of the recess portion 16 is preferably equal to or less than the particle diameter of a particle to be captured. With such depth, it is possible to prevent doublet of particles in the recess portion 16 and accumulation of another particle on a captured particle.

Here, the "particle diameter" of a particle indicates an average value of a major axis diameter and a minor axis diameter of a microparticle. Specifically, in the case of a microparticle, the particle diameter can be calculated in such a manner that a microscope is used to measure a considerable number of (e.g., 100) any microparticles using image processing software or the like to determine the average number.

For example, the depth of the recess portion 16 can be preferably two or less, more preferably one or less in the ratio to the particle diameter of a particle to be captured.

Alternatively, the depth of the recess portion 16 can be preferably two or less, more preferably one or less in the ratio to the diameter of an inscribed circle at the opening of the recess portion 16.

Furthermore, the depth of the recess portion 16 can be preferably one or less, more preferably 0.8 or less in the ratio to the height from the valley portion 14 to the mountain portion 13.

Furthermore, in a case where the opening has a circular shape in which a the three-dimensional shape of the recess portion 16 is, for example, a cylindrical shape, a circular truncated conical shape, an inverted circular truncated conical shape, a tapered shape, or an inverted tapered shape, the diameter of the recess portion 16 is preferably a size equal to or more than one time and less than two times of the particle diameter of a particle to be captured. Furthermore, in a case where the opening of the recess portion 16 has a polygonal shape of a triangular column or a column with more corners, in the case of a polygon with n sides, which is an odd number, the normal from the apex angle to the base can be regarded as the diameter and in the case of a polygon with n sides, which is an even number, the diagonal can be regarded as the diameter. If the diameter is less than one time, a single cell hardly enters into the recess portion 16. In the case of two times or more, a plurality of cells can enter into the recess portion 16.

The height from the valley portion 14 to the mountain portion 13 is preferably equal to or larger than the particle diameter of a particle to be captured. The flow speed of the liquid in the flow channel 12 increases toward a central portion. Therefore, in a case where the height of the mountain portion 13 and the valley portion 14 is smaller than the particle diameter of the particle, the flow speed to which the particle is subjected is slow even in the vicinity of the mountain portion 13. When the flow speed in the vicinity of the mountain portion 13 is slow, particles that flow subsequently tend to adhere to the particle trapped in the recess portion 16. The particles that flow subsequently also have a reduced impact energy because of slow flow speed and increasingly adhere to the captured particle, resulting in accumulation of particles.

The pitch between the mountain portions 13 can be a length equal to or more than 2 times and equal to or less than 20 times of the particle diameter of a particle to be captured. Specifically, the distance from the top portion 15 of the mountain portion 13 to a top portion 15 of an adjacent mountain portion 13 across one valley portion 14 is equal to or more than 2 times and equal to or less than 20 times of the particle diameter of a particle to be captured. In the case of less than two times, the particle can enter the valley portion 14. In the case of exceeding 20 times, depending on the height of the mountain portion 13, the wave structure becomes close to a flat structure and there is a possibility that the effect of the present technology cannot be fully exhibited.

Note that the pitch between the mountain portions 13 is more preferably a length equal to or more than 5 times and equal to or less than 15 times of the particle diameter of a particle to be captured. Within the aforementioned range, the effect to be provided by the wave structure of the present technology can be exhibited. Furthermore, in a case where the single-particle capturing apparatus of the present technology is to capture a micro-order single microparticle, a fine wave structure or recess portion needs to be formed on the substrate. The aforementioned range can be made in view also of manufacturability in that case.

Note that the right and left pitch of the mountain portion 13 may be equal and may be different.

Furthermore, regarding the flow channel 12, when the bottom surface and the top surface are parallel and the wave structure is formed on the bottom surface, the channel width of the flow channel 12 can be relatively small at the mountain portion 13 and large at the valley portion 14. With such a channel width, the particle remaining at the top portion 15 can be flown because the central laminar flow of the liquid flow is fast.

An example of sizes of the portions of the single-particle capturing apparatus described above is illustrated in Fig. 5. The single-particle capturing apparatus herein is assumed to capture a single cell or bead with a diameter size of 10 µm.

In Fig. 5, the mountain portion 13 has a width of 70 µm, the mountain portion 13 has a height of 15 µm, the top portion 15 has a width of 20 µm, the opening of the recess portion 16 has a diameter of 15 µm, the recess portion 16 has a depth of 10 µm, the draw-in passage 17 has a length of 35 µm, and the draw-in passage has a width of 3 µm.

### <2. Embodiments>

### (1) Embodiment 1

Fig. 6 illustrates a single-particle capturing apparatus of Embodiment 1. The particle to be captured by the single-particle capturing apparatus was a polystyrene bead having a diameter of 15 µm. For the substrate, polydimethylsiloxane (PDMS) was used as a material and poured into a mold, which was a master, to form a PDMS resin to produce a chip including a produced flow channel and micro wells. The PDMS substrate, which was a produced chip, was hydrophilized on the surface by direct plasma (DP) ashing with O₂: 10 cc, at 100 W, for 30 seconds, and was bonded to a cover glass in the atmosphere.

A single-particle capturing apparatus 100 manufactured by the aforementioned production method includes the flow channel 12 at a central portion of a substrate plate. The flow channel 12 includes a wave structure 31 and the recess portion 16, described above, on the top surface side, and includes an outside 18 on the bottom surface side. Thus, the wave structure 31 configures a top surface side flow channel and a bottom surface side flow channel (outside 28). An upper left port of the substrate plate is connected to the flow channel 12, and a particle-containing sample is introduced into the port. Then, a bypass 24 is installed on the right side of the substrate plate. The bypass 24 connects the top surface side flow channel and the bottom surface side flow channel. The valve 21 is installed on the bypass 24. A lower left port is a part into which the sample liquid flowing from the top surface side flow channel to the bottom surface side flow channel flows.

The particle-containing sample introduced through the upper left well can be flown into the flow channel 12 by any one of a force for introducing the particle-containing sample in the top surface side flow channel, a downstream flow force, a sample liquid flowing force generated by opening and closing of the valve 21 installed at the bypass 24, a force of sucking the sample liquid through the lower left port, and the like, or an appropriate combination thereof.

### (2) Conventional Technology Example 1

Figs. 7 and 8 illustrate a single-particle capturing apparatus of a conventional technology. The single-particle capturing apparatus was produced in a similar way to Embodiment 1 except that the wave structure was a flat surface structure.

As illustrated in Fig. 7, the single-particle capturing apparatus of the conventional technology includes the recess portion 16 on a flat surface of the flow channel 12 on the top surface side and includes the draw-in passage 17 for communication between the recess portion 16 and the outside 18. The particle-containing sample is introduced into the flow channel 12 on the top surface side in a direction of the upper left arrow of Fig. 7, the sample liquid flow moves to the outside 18 through a tube 25 that includes an increment 26 and couples the flow channel 12 on the top surface side to the outside 18, and is discharged in a direction of the lower left arrow of Fig. 7.

A bead capture experiment was conducted using the single-particle capturing apparatus of the aforementioned conventional technology of Fig. 7.

First, in order to prepare a particle-containing sample, a liquid concentrate of polystyrene bead having a diameter of 20 µm was diluted 1000 times.

The single-particle capturing apparatus was mounted on a jig, the bead diluted liquid was inserted by a syringe pump through an inlet port (part indicated by the upper left arrow of Fig. 7), and the pressure in the flow channel was reduced by a suction pump through an outlet port (part indicated by the lower left arrow of Fig. 7) for easy liquid flow.

Suction was started with - 10 kPa, and the suction amount was gradually increased to - 35 kPa. The suction pressure was adjusted by compression of the tube 25 about 0.7 mm using the increment 26 with 1.1 mm. The first flow speed at which the liquid is supplied by the syringe pump was set to 50 µL/min, the flow speed was gradually increased to 100 µL/min.

As illustrated in Fig. 7, a bead 102 began being captured in the recess portion 16 of the single-particle capturing apparatus of the conventional technology. However, when the bead diluted liquid was continuously flown, as illustrated in Fig. 8, a phenomenon was seen in which two or more beads entered into the recess portion 16 or another bead adhered to a captured bead.

### (3) Conventional Technology Example 2 and Embodiment 2

A single-particle capturing apparatus including a wave structure was indicated as Embodiment 2 at 9A of Fig. 9. A single-particle capturing apparatus including a flat surface was indicated at 9C as Conventional Technology Example 2. A single-particle capturing apparatus including a flat surface structure on the left side and a wave structure on the right side of the dotted line was indicated at 9B.

As the particle-containing sample, a liquid concentrate of polystyrene bead having a diameter of 15 µm was diluted 1000 times, and a bead diluted liquid was prepared to have a bead concentration of 1.7 µl and Tween20 of 0.05%.

The bead diluted liquid was flown to the single-particle capturing apparatuses indicated at 9A, 9B, and 9C. In the case of 9A, it was observed that one or two beads were captured in each of the recess portions at the top portion of the wave structure.

In the case of 9C, it was observed that beads were captured in the recess portions present on the flat surface structure and other beads were adhered to the captured beads and accumulated.

In the case of 9B, it was observed that, although the flat surface structure and the wave structure were arranged on the same flow channel, beads were accumulated in the recess portions of the flat surface structure but beads were not accumulated in the recess portions of the wave structure.

Note that the sizes of the recess portions are indicated at 10A and 10B of Fig. 10.

The recess portion of the wave structure of 10A has a diameter of 15 µm and a depth of 25 µm. The depth was larger than the diameter of 15 µm of the bead particle diameter, and two beads were captured in the recess portion.

The recess portion of the flat surface structure of 10B had a diameter of 15 µm and a depth of 25 µm. Not only a plurality of beads was captured in the recess portion, but also other beads were adhered to and accumulated on the captured beads.

### (4) Embodiment 3

Fig. 11 illustrates the single-particle capturing apparatus of the present technology at 11A and 11B. The single-particle capturing apparatus was produced in a similar way to Embodiment 1. Note that the recess portion had a depth of 10 µm.

The single-particle capturing apparatus was mounted on a jig, the bead diluted liquid was inserted by a syringe pump through an inlet port, and the pressure in the flow channel was reduced by a suction pump through an outlet port for easy liquid flow.

The operation conditions are described below.

Syringe pump flow speed: 6 mL/h (= 100 mL/min)
Suction pressure: maintained at - 10 kPa (bypass tube compression amount: 0.7 mm)
Bead flow rate: 0.28/sec
Inside flow speed: 0.6 ml/h = 0.167 ul/sec

Furthermore, as the particle-containing sample, a liquid concentrate of polystyrene bead having a diameter of 15 µm was diluted 1000 times, and a bead diluted liquid was prepared to have a bead concentration of 1.7 µl and Tween20 of 0.05%.

When the bead diluted liquid was flown to the single-particle capturing apparatus, a single bead stably entered the recess portion as indicated at 11A. The reason for this is considered to be the fact that, due to presence of the flow channel side surface of the wave structure arranged in the flow channel and the recess portion arranged at the top portion of the mountain portion, even if a first bead is captured in the recess portion and a next bead is sucked to the recess portion and attempts to adhere to the captured bead, the bead that attempts to adhere contacts a subsequent bead or the liquid flow and is flown downstream because of the flow speed at the top portion which is faster by about 20% than the flow speed at the side wall. Accordingly, the captured beads hardly make doublet.

However, when the beads are supplied excessively, as indicated at 11B, a phenomenon was seen in which the beads were accumulated along the wave structure. The accumulation of the beads was able to be re-dispersed when the suction pressure was released to change the flow of the bead diluted liquid.

### (5) Embodiment 4

### [Consideration 1 for dispersion of particles by pulsation]

Fig. 12 illustrates the single-particle capturing apparatus including the recess portion (flat surface well) formed on the flat surface on the left side of the dotted line and the recess portion (wave-shaped well) formed on the wave structure on the right side.

When the bead diluted liquid was supplied to the single-particle capturing apparatus, as illustrated in the photograph "ALIGNMENT" at the top of Fig. 12, another particle began adhering to the captured particle in the recess portion at the flat surface well. When the bead diluted liquid was further supplied continuously, as illustrated in the photograph "ACCUMULATION" of Fig. 12, the beads began accumulating at the flat surface well and also accumulating at the wave-shaped well.

Here, when depressurization and pressurization were repeatedly applied to the flow channel of Fig. 12 to pulsate the flow of the bead diluted liquid, as illustrated in the photograph "PULSATION" of Fig. 12, it was observed that the accumulated beads were moved.

However, although the bead dispersion function was provided by pulsation, a subsequent bead was adhered and aggregated in a bead return process at the flat surface well. Eventually, a similar accumulated state was made before and after the pulsation (photograph "WASH OUT" of Fig. 12). The particle dispersion effect by pulsation gave more favorable results at the wave-shaped well than at the flat surface well.

### (6) Embodiment 5

### [Consideration 2 for dispersion of particles by pulsation]

The particle dispersion effect by pulsation at a central portion and an end portion of a flow channel of a single-particle capturing apparatus including a wave structure was considered.

As illustrated in Fig. 13, when looking at the recess portion of the wave structure at a central portion of the flow channel of the single-particle capturing apparatus 100, when the bead diluted liquid was supplied excessively, accumulation of beads was observed. When looking at the recess portion of the wave structure at an end portion of the flow channel, a great number of points where beads were not captured were observed.

Next, depressurization and pressurization were repeatedly applied to the flow channel to pulsate the bead diluted liquid. Then, the beads were re-dispersed and the accumulation at the bead accumulation points at the central portion was eliminated (washed out). A single bead was captured in the recess portion at the end portion where beads had not been captured (realignment).

From the above, it has become apparent that, although excessive bead supply results in accumulation along the shape of the flow channel, when the pressure of the particle-containing sample is periodically released, the accumulated particles can be re-dispersed, a particle can be captured in an empty recess portion during operation and particles can be flown and moved.

### (7) Embodiment 6

### [Example of single-particle capturing apparatus in high-density arrangement with independent suction path and liquid supply path]

As illustrated in Fig. 14, a single-particle capturing apparatus including a flow channel having a lateral U-shape, a wave structure and a recess portion on an inner side of the lateral U-shape, an outside for suction at a center on an inner side of the lateral U shape, and a draw-in passage through which the recess portion communicates with the outside was produced. The sample was flown in the direction indicated by thin arrows, and suction was conducted in the direction indicated by a bold arrow.

In this way, a single-particle capturing apparatus in high-density arrangement including a wave structure in which a side surface of one flow channel drawn with a single stroke of brush is a sine wave, a recess portion is arranged at the top portion of the wave structure, a draw-in passage is arranged on the bottom surface of the recess portion, the wave structure and the recess portion are formed on right and left side surfaces and upper and lower side surfaces of the flow channel can be produced. In this way, when a plurality of one-dimensional flow channels is arranged in parallel, it is possible to increase the number of particles captured.

Note that in the present technology, curvature or bent is not limited to a U-shape, but includes any curved or bent patterns including a C-shape, an E-shape, an H-shape, an I-shape, an L-shape, an M-shape, an N-shape, an S-shape, a T-shape, a V-shape, a W-shape, an X-shape, a Y-shape, a meandering-shape, a helical shape, and the like.

### (8) Embodiment 7

### [Example of single-particle capturing apparatus in three-dimensional high-density arrangement]

As schematically illustrated in Fig. 15, a three-dimensional high-density arrangement can also be produced. 15C is an example in which the mountain portion of the wave structure has a circular truncated conical shape and the recess portion is formed at the top portion. An example in which the arrangement of the circular truncated cones densely aligned longitudinally and laterally is viewed from top and side is 15A, and an example in which the arrangement with the rows displaced is viewed from top and side is 15B.

In this way, when circular truncated cones including the recess portion at the top portion are densely arranged on the flat surface, a one-dimensional wave flow channel can be deployed on the plane surface and arranged. Then, when the flow is configured to occur from one direction on the flat surface, the effect equivalent to the one-dimensional wave flow channel can be obtained.

### (9) Embodiment 8

### [Example of parallel flow channels]

Fig. 16 illustrates a single-particle capturing apparatus in which flow channels are arranged in parallel. At 16A, flow channels are arranged in three rows. At 16B, the structure of a left end of the flow channel is enlarged to be seen. At 16C, the structure of a right end of the flow channel is enlarged to be seen.

As illustrated by the perspective views of 16E and 16F, each flow channel includes a wave structure, a recess portion, and a draw-in passage on both sides (upper and lower inner sides of the flow channel).

The particle-containing sample is supplied from an introduction portion on a left side of 16A and passes through each flow channel. The flow is split into two at a right end and the sample liquid flows outside such that positive pressure occurs because of the presence of the draw-in passage and the particle is captured in each recess portion. The photograph before capturing of beads is 16D, and the photograph after capturing is 16G. It was observed that the beads were captured in the recess portions on both sides of the flow channel (16G).

### (10) Embodiment 9

### [Example of mounting technology for self-alignment of IC chip]

A single-particle capturing apparatus of the present technology was produced from an on-chip IC (SoC: System-on-Silicon) substrate.

As a particle to be captured, those made by cutting a high-density IC chip produced by a semiconductor process on a silicon wafer into a 100 µm square using a dicer from above the wafer were prepared. Depending on the cut-out size and the width of a cutting margin, the number of IC chips to be prepared is seven million from a 300 mm wafer.

Conventionally, mounting the above in an aligned matter state at equal intervals and narrow pitches by self-alignment, there is a limitation with a chip mounter (0.4 mm x 0.2 mm square).

However, microchips at narrow pitches can be mounted along at equal intervals by utilizing a self-assembly method using a flow channel including the wave structure 31 according to the present technology.

The IC chip arranged in the recess portion at the top portion of the wave structure can be combined and wired with a different chip in a subsequent process.

Furthermore, a substrate integrated with a flow channel substrate can be produced such that a different electric circuit substrate is preliminarily made near the top portion of the wave-shaped flow channel, and wiring is performed by a wire bonder or the like when the IC chip is trapped in the recess portion for extensive arrangement.

Furthermore, an on-chip IC devise can be produced efficiently by cutting out.

### (11) Embodiment 10

### [Application to production of micro LED display]

A single-particle capturing apparatus illustrated in Fig. 17 was produced. Three lanes of independent flow channels including the wave structure are prepared, different micro LED chips are dispersed in the liquid of the lanes, the liquid is supplied in a direction of a left side arrow of Fig. 17, and a red LED, a blue LED, and a green LED are flown in the respective lanes. Thus, the LEDs can be mounted at equal intervals at 150 µm pitches.

The LED chips captured at the top portions of the wave structure 31 can be used as a micro LED display by being wired by a wire bonder to the capturing recess portions and global electrodes 27 arranged on a lower side of the recess portions.

Furthermore, similarly, in the case of an active drive-type display, e.g., an organic EL, this mounting technology can be applied and IC chips on which IC circuits, which are currently produced of polysilicon, are independently made relative to pixels can be mounted at equal interval pitches. Therefore, an active matrix polysilicon circuit that is expensive and has poor yield can be replaced by a stably operable IC chip.

### <3. Single-particle capturing system>

The single-particle capturing system of the present technology includes a liquid supply unit in the single-particle capturing apparatus.

Fig. 18 illustrates an example of a single-particle capturing system 101.

A single-particle capturing unit 102 is coupled to a liquid supply unit 103 via the valve 21. The liquid supply unit 103 supplies the particle-containing sample to the single-particle capturing unit 102. The flow of the sample can be controlled by opening and closing of the valve 21. The control can be performed by a liquid supply control unit 106. A control program may be included in a computer to enable automatic liquid supply control. Control of liquid supply can not only flow/stop the sample, but also generate a backward flow and even a pulsatile flow that changes the flow at regular intervals.

Furthermore, the single-particle capturing system 101 may include a single-particle observing unit 105. The single-particle observing unit 105 is not particularly limited. However, the flow channel and a state in which particles flow and are captured may be zoomed in under an electron microscope or the like so as to be observed by the naked eye or may be subject to data processing by an image processing apparatus or the like without the naked eye. The results of the observation in this case can be fed back to the liquid supply control unit 106, and the flow of the sample can be further controlled.

Moreover, the single-particle capturing system 101 may include a waste liquid unit 104 on a downstream side. The sample liquid including a reduced particle content can be recovered as a waste liquid. A valve or pump may be further included on an upstream side or a downstream side of the waste liquid unit 104 such that a suction force is exerted on the flow channel of the single-particle capturing unit 102.

### <4. Single-particle capturing method>

A single-particle capturing method of the present technology is a method in which a specimen containing a particle to be captured is supplied to the single-particle capturing apparatus and the specimen while being supplied is sucked to the outside from the recess portion via the draw-in passage such that the particle to be captured is captured.

As described above, the supplied liquid can be flown backward. Forward flow and backward flow are repeatedly generated to disperse the accumulated particles such that the particles enter all the recess portions.

Note that the present technology may adopt the configuration described below.
[1] A single-particle capturing apparatus including:
   a flow channel on a substrate,
   a wave structure with a mountain portion and a valley portion on the flow channel, and
   a recess portion at a top portion of the mountain portion, the recess portion including a draw-in passage.
[2] The single-particle capturing apparatus according to [1], in which the recess portion has a depth equal to or smaller than a particle diameter of a particle to be captured.
[3] The single-particle capturing apparatus according to [1] or [2], in which a diameter of the recess portion is a size equal to or more than one time and less than two times of a particle diameter of a particle to be captured.
[4] The single-particle capturing apparatus according to any of [1] to [3], in which a height from the valley portion to the mountain portion is equal to or larger than a particle diameter of a particle to be captured.
[5] The single-particle capturing apparatus according to any of [1] to [4], in which a pitch between the mountain portions is a length equal to or more than 2 times and equal to or less than 20 times of a particle diameter of a particle to be captured.
[6] The single-particle capturing apparatus according to any of [1] to [5], in which a channel width of the flow channel is relatively small at the mountain portion and relatively large at the valley portion.
[7] The single-particle capturing apparatus according to any of [1] to [6], in which the draw-in passage makes communication between the recess portion and the outside.
[8] The single-particle capturing apparatus according to [7], in which the outside is coupled to the flow channel.
[9] The single-particle capturing apparatus according to any of [1] to [8], in which a plurality of the mountain portions and a plurality of the valley portions are aligned on a bottom surface of the flow channel.
[10] The single-particle capturing apparatus according to [1] to [9], in which the flow channel and the wave structure are curved or bent.
[11] The single-particle capturing apparatus according to [10], in which the flow channel and the wave structure are curved in a U-shape, and an inner side of the U-shape is the outside.
[12] A single-particle capturing system including:
   a single-particle capturing unit including
      a flow channel on a substrate,
      a wave structure with a mountain portion and a valley portion, on the flow channel, and
      a recess portion at a top portion of the mountain portion, the recess portion including a draw-in passage; and
   a liquid supply unit.
[13] The single-particle capturing system according to [12], in which the flow channel includes a valve.
[14] The single-particle capturing system according to [12] or [13], further including a waste liquid unit.
[15] The single-particle capturing system according to any of [12] to [14], further including a single-particle capturing observing unit configured to observe the single-particle capturing unit.
[16] The single-particle capturing system according to any of [12] to [15], further including a liquid supply control unit configured to control the liquid supply unit.
[17] A single-particle capturing method, in which a specimen containing a particle to be captured is supplied to a single-particle capturing apparatus including:
   a flow channel on a substrate,
   the flow channel including a wave structure with a mountain portion and a valley portion, and
   a recess portion at a top portion of the mountain portion, the recess portion including a draw-in passage, and
   the specimen is, while being supplied, sucked from the recess portion to an outside via the draw-in passage such that the particle to be captured is captured.
[18] The single-particle capturing method according to [17], including flowing the supplied liquid backward.

### REFERENCE SIGNS LIST

- 11: Substrate
- 12: Flow channel
- 13: Mountain portion
- 14: Valley portion
- 15: Top portion
- 16: Recess portion
- 17: Draw-in passage
- 18: Outside
- 19: Top surface
- 21: Valve
- 22: Liquid flow direction
- 23: Draw-in by positive pressure
- 24: Bypass
- 25: Tube
- 26: Increment
- 27: Global wiring
- 31: Wave structure
- 100: Single-particle capturing apparatus
- 101: Single-particle capturing system
- 102: Bead
- 103: Liquid supply unit
- 104: Waste liquid unit
- 105: Single-particle observing unit
- 106: Liquid supply control unit

## Claims

1. A single-particle capturing apparatus comprising:
a flow channel on a substrate,
the single-particle capturing apparatus including
a wave structure with a mountain portion and a valley portion on the flow channel, and
a recess portion at a top portion of the mountain portion, the recess portion including a draw-in passage.

2. The single-particle capturing apparatus according to claim 1, wherein the recess portion has a depth equal to or smaller than a particle diameter of a particle to be captured.

3. The single-particle capturing apparatus according to claim 1, wherein a diameter of the recess portion is a size equal to or more than one time and less than two times of a particle diameter of a particle to be captured.

4. The single-particle capturing apparatus according to claim 1, wherein a height from the valley portion to the mountain portion is equal to or larger than a particle diameter of a particle to be captured.

5. The single-particle capturing apparatus according to claim 1, wherein a pitch between the mountain portions is a length equal to or more than 2 times and equal to or less than 20 times of a particle diameter of a particle to be captured.

6. The single-particle capturing apparatus according to claim 1, wherein a channel width of the flow channel is relatively small at the mountain portion and relatively large at the valley portion.

7. The single-particle capturing apparatus according to claim 1, wherein the draw-in passage makes communication between the recess portion and an outside.

8. The single-particle capturing apparatus according to claim 7, wherein the outside is coupled to the flow channel.

9. The single-particle capturing apparatus according to claim 1, wherein a plurality of the mountain portions and a plurality of the valley portions are aligned on a bottom surface of the flow channel.

10. The single-particle capturing apparatus according to claim 1, wherein the flow channel and the wave structure are curved or bent.

11. The single-particle capturing apparatus according to claim 7, wherein the flow channel and the wave structure are curved in a U-shape, and an inner side of the U-shape is the outside.

12. A single-particle capturing system comprising:
a single-particle capturing unit including
a flow channel on a substrate,
a wave structure with a mountain portion and a valley portion on the flow channel, and
a recess portion at a top portion of the mountain portion, the recess portion including a draw-in passage; and
a liquid supply unit.

13. The single-particle capturing system according to claim 12, wherein the flow channel includes a valve.

14. A single-particle capturing method, wherein a specimen containing a particle to be captured is supplied to a single-particle capturing apparatus including:
a flow channel on a substrate,
the flow channel including a wave structure with a mountain portion and a valley portion, and
a recess portion at a top portion of the mountain portion, the recess portion including a draw-in passage, and
the specimen is, while being supplied, sucked from the recess portion to an outside via the draw-in passage such that the particle to be captured is captured.

15. The single-particle capturing method according to claim 14, comprising flowing the supplied liquid backward.

## Patentansprüche

1. Einzelpartikelerfassungsvorrichtung, umfassend:
einen Strömungskanal auf einem Substrat,
wobei die Einzelpartikelerfassungsvorrichtung umfasst
eine Wellenstruktur mit einem Bergabschnitt und einem Talabschnitt auf dem Strömungskanal, und
einen Vertiefungsabschnitt an einem oberen Abschnitt des Bergabschnitts, wobei der Vertiefungsabschnitt einen Einzugsdurchgang aufweist.

2. Einzelpartikelerfassungsvorrichtung nach Anspruch 1, wobei der Vertiefungsabschnitt eine Tiefe von gleich oder kleiner als einen Partikeldurchmesser eines zu erfassenden Partikels aufweist.

3. Einzelpartikelerfassungsvorrichtung nach Anspruch 1, wobei ein Durchmesser des Vertiefungsabschnitts eine Größe hat, die gleich oder mehr als einmal und weniger als zweimal so groß wie ein Partikeldurchmesser eines zu erfassenden Partikels ist.

4. Einzelpartikelerfassungsvorrichtung nach Anspruch 1, wobei die Höhe vom Talabschnitt zum Bergabschnitt gleich oder größer als ein Partikeldurchmesser eines zu erfassenden Partikels ist.

5. Einzelpartikelerfassungsvorrichtung nach Anspruch 1, wobei ein Abstand zwischen den Bergabschnitten eine Länge von gleich oder mehr als das 2-Fache und gleich oder weniger als das 20-Fache des Partikeldurchmessers eines einzufangenden Teilchens aufweist.

6. Einzelpartikelerfassungsvorrichtung nach Anspruch 1, wobei eine Kanalbreite des Strömungskanals am Bergabschnitt relativ klein und am Talabschnitt relativ groß ist.

7. Einzelpartikelerfassungsvorrichtung nach Anspruch 1, wobei der Einzugsdurchgang eine Verbindung zwischen dem Vertiefungsabschnitt und einer Außenseite herstellt.

8. Einzelpartikelerfassungsvorrichtung nach Anspruch 7, wobei die Außenseite mit dem Strömungskanal gekoppelt ist.

9. Einzelpartikelerfassungsvorrichtung nach Anspruch 1, wobei eine Vielzahl der Bergabschnitte und eine Vielzahl der Talabschnitte auf einer Bodenfläche des Strömungskanals ausgerichtet sind.

10. Einzelpartikelerfassungsvorrichtung nach Anspruch 1, wobei der Strömungskanal und die Wellenstruktur gekrümmt oder gebogen sind.

11. Einzelpartikelerfassungsvorrichtung nach Anspruch 7, wobei der Strömungskanal und die Wellenstruktur U-förmig gekrümmt sind und eine Innenseite der U-Form die Außenseite ist.

12. Einzelpartikelerfassungssystem, umfassend:
eine Einzelpartikelerfassungseinheit, die Folgendes aufweist:
einen Strömungskanal auf einem Substrat,
eine Wellenstruktur mit einem Bergabschnitt und
einen Talabschnitt auf dem Strömungskanal und
einen Vertiefungsabschnitt an einem oberen Abschnitt des Bergabschnitts, wobei der Vertiefungsabschnitt einen Einzugsdurchgang aufweist; und
eine Flüssigkeitszuführeinheit.

13. Einzelpartikelerfassungssystem nach Anspruch 12, wobei der Strömungskanal ein Ventil aufweist.

14. Verfahren zur Erfassung von Einzelpartikeln, wobei eine Probe, die ein zu erfassendes Partikel enthält, einer Einzelpartikelerfassungsvorrichtung zugeführt wird, die Folgendes aufweist:
einen Strömungskanal auf einem Substrat,
den Strömungskanal, der eine Wellenstruktur mit einem Bergabschnitt und einem Talabschnitt aufweist, und
einen Vertiefungsabschnitt an einem oberen Abschnitt des Bergabschnitts, wobei der Vertiefungsabschnitt einen Einzugsdurchgang aufweist und
die Probe beim Zuführen aus dem Vertiefungsabschnitt über den Einzugsdurchgang an eine Außenseite gesaugt wird, sodass das zu erfassende Partikel erfasst wird.

15. Verfahren zur Erfassung von Einzelpartikeln nach Anspruch 14, umfassend ein Rückwärtsfließen der zugeführten Flüssigkeit.

## Revendications

1. Appareil de capture de particule unique comprenant :
un canal d'écoulement sur un substrat,
l'appareil de capture de particule unique comportant
une structure ondulée avec une partie de montagne et une partie de vallée sur le canal d'écoulement, et
une partie creusée au niveau d'une partie supérieure de la partie de montagne, la partie creusée comportant un passage d'entraînement.

2. Appareil de capture de particule unique selon la revendication 1, dans lequel la partie creusée a une profondeur égale ou inférieure à un diamètre de particule d'une particule devant être capturée.

3. Appareil de capture de particule unique selon la revendication 1, dans lequel un diamètre de la partie creusée est une taille égale ou supérieure à une fois et inférieure à deux fois un diamètre de particule d'une particule devant être capturée.

4. Appareil de capture de particule unique selon la revendication 1, dans lequel une hauteur de la partie de vallée à la partie de montagne est égale ou supérieure à un diamètre de particule d'une particule devant être capturée.

5. Appareil de capture de particule unique selon la revendication 1, dans lequel un pas entre les parties de montagne est une longueur égale ou supérieure à 2 fois et égale ou inférieure à 20 fois un diamètre de particule d'une particule devant être capturée.

6. Appareil de capture de particule unique selon la revendication 1, dans lequel une largeur de canal du canal d'écoulement est relativement petite au niveau de la partie de montagne et relativement grande au niveau de la partie de vallée.

7. Appareil de capture de particule unique selon la revendication 1, dans lequel le passage d'entraînement fait communication entre la partie creusée et un extérieur.

8. Appareil de capture de particule unique selon la revendication 7, dans lequel l'extérieur est couplé au canal d'écoulement.

9. Appareil de capture de particule unique selon la revendication 1, dans lequel une pluralité des parties de montagne et une pluralité des parties de vallée sont alignées sur une surface inférieure du canal d'écoulement.

10. Appareil de capture de particule unique selon la revendication 1, dans lequel le canal d'écoulement et la structure ondulée sont incurvés ou pliés.

11. Appareil de capture de particule unique selon la revendication 7, dans lequel le canal d'écoulement et la structure ondulée sont incurvés en forme de U, et un côté interne de la forme en U est l'extérieur.

12. Système de capture de particule unique comprenant :
une unité de capture de particule unique comportant
un canal d'écoulement sur un substrat,
une structure ondulée avec une partie de montagne et une partie de vallée sur le canal d'écoulement, et
une partie creusée au niveau d'une partie supérieure de la partie de montagne, la partie creusée comportant un passage d'entraînement ; et
une unité de fourniture de liquide.

13. Système de capture de particule unique selon la revendication 12, dans lequel le canal d'écoulement comporte une vanne.

14. Procédé de capture de particule unique, dans lequel un échantillon contenant une particule devant être capturée est fourni à un appareil de capture de particule unique comportant :
un canal d'écoulement sur un substrat,
le canal d'écoulement comportant une structure ondulée avec une partie de montagne et une partie de vallée, et
une partie creusée au niveau d'une partie supérieure de la partie de montagne, la partie creusée comportant un passage d'entraînement, et
l'échantillon est, pendant qu'il est fourni, aspiré depuis la partie creusée jusqu'à un extérieur par le biais du passage d'entraînement de telle sorte que la particule devant être capturée est capturée.

15. Procédé de capture de particule unique selon la revendication 14, comprenant l'écoulement du liquide fourni en arrière.
